# EUROPEAN PATENT SPECIFICATION

(11) **EP 0 884 524 B1**
(45) Date of publication and mention of the grant of the patent: **16.07.2003**
(21) Application number: 98108119.3
(22) Date of filing: 05.05.1998
(51) Int. Cl.: F21V 8/00

(54) **A device for lighting and use of such device**
Beleuchtungseinrichtung und deren Anwendung
Dispositif d' éclairage et son application

(30) Priority: 05.05.1997 DK 19097
(43) Date of publication of application: 16.12.1998
(73) Proprietor: Bendixen Neon ApS, 8800 Viborg (DK)
(72) Inventor: Kjellerup, Knud, DK-8800 Viborg (DK); Sorensen, Kim Albech, DK-8800 Viborg (DK)
(74) Representative: Nielsen, Leif

(56) References cited:
- DE-A- 4 404 247
- GB-A- 2 238 109

## Description

The present invention relates to a device for lighting, said device comprising a translucent element and a reflector.

EP-B1-0 562 802 discloses such a device. The translucent element has a cross section that is not rectangular viewed in parallel with a direction for light from the light source. The translucent element is made from acrylic. A front side or a back side of the translucent element is coated with a reflecting layer and light from the light source is input to the translucent element from a side surface of the translucent element. The translucent element is preferably wedge-shaped, and light from the light source is directed through a foot of the wedge, is reflected off the wedge surface that is coated with the reflecting layer, and is output through the other surface of the wedge. The device is primarily intended for use in liquid crystal displays.

The device has certain drawbacks. Because of the wedge-shape, it requires great precision to achieve the desired distribution of light, and the requirement for the treatment of the translucent element and the requirement for the quality of the translucent element become very great. Furthermore, the light source is a common fluorescent light source which is input to the translucent material from the translucent element. This makes great demands on the treatment of the surface of the foot of the wedge. Finally, the dimensions of the known device are considerable, if the device is to be used for large applications like facade signs.

Another document to consider is GB-A-2 238 109.

It is the purpose with the present invention to provide a device capable of providing lighting out through the back side of a translucent element while at the same time the translucent element displays a considerable reduction of the depth.

This purpose is achieved with a first device characterised in that the device comprises a translucent material with a front side, a back side and side surfaces, that the reflector is in contact with the front side, that the light rays are intended to be sent forwards towards the front of the translucent element, that the light rays are intended to be reflected off the reflector and to be emitted through the back side of the translucent element.

The purpose is further achieved with a second device for lighting, said device comprising a translucent element and a reflector situated at a distance from the translucent element, and wherein a light source is intended to send light into a space between the translucent element and an inner side of the reflector, said device being characterised in that the light is intended to be sent forwards towards the inner side of the reflector, that the light is intended to be reflected off the inner side of the reflector and to be emitted through the back side of the translucent element.

A preferred embodiment for the device is characterised in that the device at least comprises a first translucent element and a second translucent element, that the first translucent element comprises the front side and the back side, that the front side and the back side are plane, that the reflector is in contact with the front side of the first translucent element, that a front side of the second translucent element contacts the back side of the first translucent element, and that a back side of the second translucent element has a non-plane surface.

By making lighting in the translucent element as indicated above, it is possible to achieve a sufficient and evenly distributed lighting through the back side of the translucent element while at the same time the depth of the translucent element becomes very small.

A further preferred embodiment of the invention is characterised in that light is transmitted to the device through an optical fibre, that a tip of the optical fibre is fitted in the first translucent element, and that the tip of the optical fibre is directed towards the front of the first translucent element

Use of an optical fibre for transmitting light from the light source to the translucent element makes it possible to situate the light source at a distance from the device. As the tip of the optical fibre is fitted inside the translucent element, the requirement for the surfaces of the translucent element is considerably less, as there is no risk of light from the light source being reflected when the light is input to the translucent element.

The invention will now be explained with reference to the accompanying drawing, in which
- Fig. 1: is a sectional view through a first embodiment of the device according to the invention,
- Fig. 2: is a sectional view through a second embodiment of the device according to the invention, and
- Fig. 3: is a view of a device according to the invention for use as a sign for a facade.

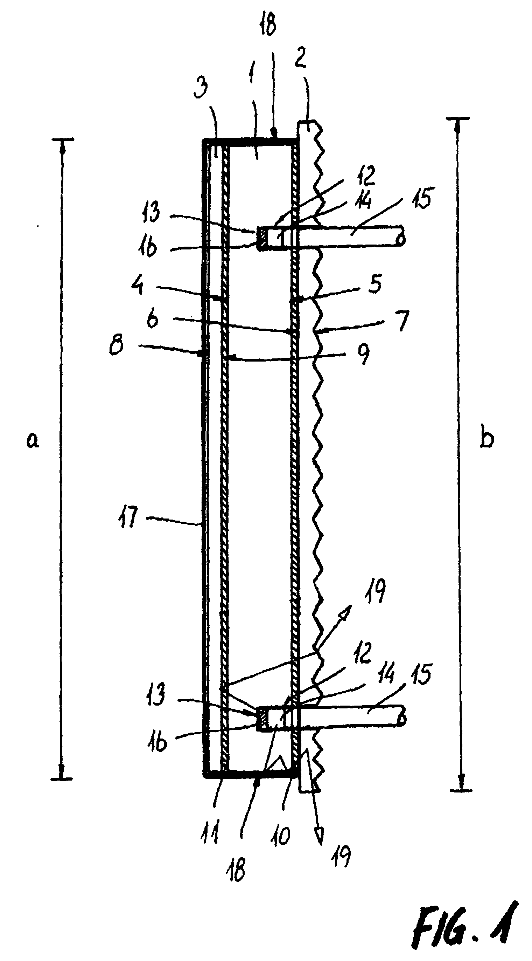

Fig. 1 shows a first embodiment of the device according to the invention. The device shown in the Figure is intended for a sign for providing corona effect on a wall, such as a sign on the facade of a building. The device comprises a first translucent element 1, and a second translucent element 2 and a third translucent element 3. The translucent element 1 has a front side 4 and a back side 5. The translucent element 2 has a front side 6 which is in contact with and secured to the back side 5 of the translucent element 1 by means of co-polymerisation which is made from a layer 10 of polymetyl methacrylate, also named PMMA, between the back side 4 of the translucent element 1 and the front side 6 of the translucent element 2. The translucent element 3 has a front side 8 and a back side 9 which are in contact with and secured to the front side 4 of the translucent element 1 also by means of co-polymerisation, which is also made from a layer 11 of polymetyl methacrylate, also named PMMA, between the front side 4 of the translucent element 1 and the back side 9 of the translucent element 2.

The translucent element 1 is provided with holes 12 with a bottom 13. A tip 14 of the optical fibres 15 is fitted in the holes 12. The tip 14 of the optical fibres 15 is secured to the bottom 13 of the holes 12 by means of optical adhesive 16. The tip 14 of the optical fibres 15 has been stripped of insulation so that light may be directed sideways out from the tip 14 of the optical fibres 15. The tip 14 of the optical fibres 15 is fitted in such a way that the optical fibres 15 extend about halfway into the translucent element 1. It is possible to fit the tip 14 of the optical fibres 15 at other depths of the translucent element 1. Also, it will be possible to omit using optical adhesive 16 or to omit stripping the tip 14 of the optical fibres 15 of insulation.

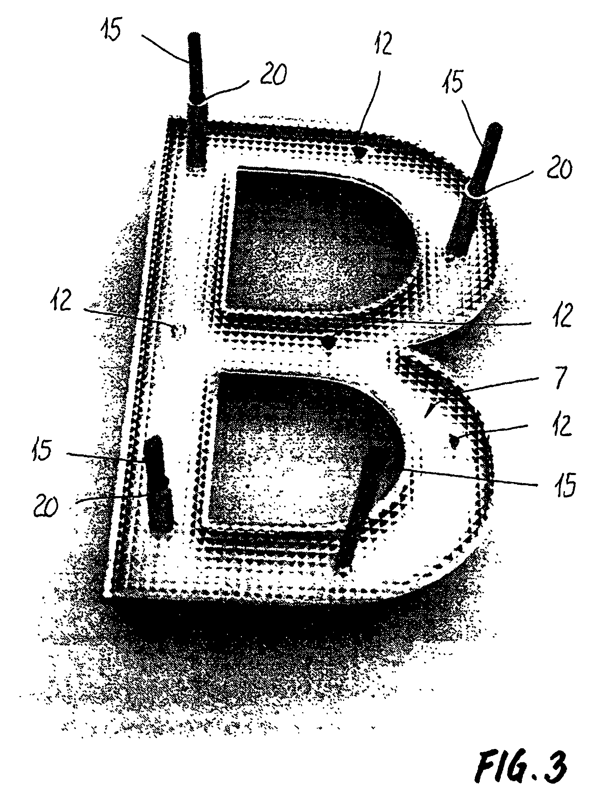

In the shown embodiment, the translucent element 1 has a plane front side 4 and a plane back side 5. In the shown embodiment, the translucent element 3 also has a plane front side 8 and a plane back side 9. The translucent element 3 forms a reflecting layer. In the shown embodiment, the translucent element 2 has a plane front side 6 and a non-plane back side 7 of a pyramid structure (see Fig. 3). The pyramid structure is ideally suited for diffusing the light when it is emitted through the back side 7 of the translucent element 2.

In an alternative embodiment, the back side 7 of the translucent element 2 is provided with other means than the pyramid structure for diffusing the light, when it is emitted through the back side 7. Other means may be e.g. a dispersion film or use of white acrylic instead of translucent acrylic. In yet another alternative embodiment, the front side 8 of the translucent element 3 is also non-plane. In a further alternative embodiment, the back side 5 of the translucent element 1 is non-plane and the translucent element 2 has been omitted, and/or the front side 4 of the translucent element 1 is non-plane and/or the translucent element 3 has been omitted.

A cover 17 covers the front side 8 of the translucent element 3 and the side surfaces 18 of the translucent element 1. The cover 17 is made from a non-translucent material. In the shown embodiment, the cover 17 consists of a shell of metal formed like the translucent element 1 and the translucent element 3. The cover 17 may be made from other materials just as the cover 17 may consist of a coating applied to the front side 8 of the translucent element 3 and the sides surfaces 18 of the translucent element 1. The cover 17 extends over a distance a. The translucent element 2 extends beyond the side surfaces 18 of the translucent element 1 over a distance b which equals or, as shown, is greater than a.

With the embodiment of the device according to the invention shown in Fig. 1, corona effect is achieved in the following way. Light 19 is transmitted from an external light source through the optical fibres 15 to the tip 14 of the optical fibres 15. The light 19 is output through the tip 14 of the optical fibres 15 partly forwards through the optical adhesive 16 and partly sideways. The light 19 is directed through the translucent element 1 forwards towards the translucent element 3 and sideways towards the side surfaces 18 of the translucent element 1. The light 19 is primarily reflected off the back side 9 of the translucent element 3, but is also reflected inside the translucent element 3. The light 19 is then directed backwards through the translucent element 1 towards the back side 5 of the translucent element 1. The light 19 is then directed through the translucent element 2 and emitted from the device through the back side 7 of the translucent element 2. The light 19 which is emitted through the back side 7 of the translucent element 2 will provide a corona effect for the shape of the cover 17 in a plane perpendicular to the plane of the paper.

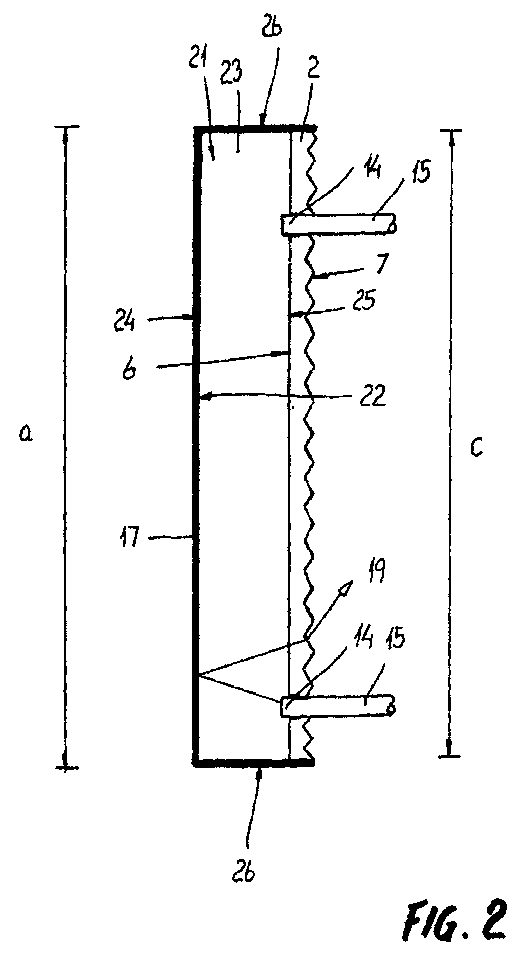

Fig. 2 shows a second embodiment of the device according to the invention. The device that is shown in the Figure is also intended for a sign for providing a corona effect on a wall, such as a sign on the facade of a building. The device only comprises the second translucent element 2. In the shown embodiment, the translucent element 2 also has a plane front side 6 and a non-plane back side 7 also of a pyramid structure (see Fig. 3). The pyramid structure is ideally suited for diffusing the light when it is emitted through the back side 7 of the translucent element 2. In an alternative embodiment, the back side 7 of the translucent element 2 is provided with other means than the pyramid structure for diffusing the light when it is emitted through the back side 7. Other means may be e.g. a dispersion film or use of white acrylic instead of transparent acrylic.

A cover 17 covers a space 21 between the translucent element 2 and the inner side 22 of the cover 17. The space may be empty or filled wholly or partly by a translucent element 23 as in the shown embodiment. If the space is filled by a translucent element 23, a back side 25 of the translucent element 23 is in contact with the front side 6 of the translucent element 2. The cover 17 is made from a non-translucent material. In the shown embodiment, the cover 17 consists of a shell of metal. The cover 17 may be made from other materials just as the cover 17 may consist of a coating applied to a front side 24 of the translucent element 23 and side surfaces 26 of the translucent element 23 and the translucent element 2, respectively. The cover 17 extends over a distance a. The translucent element 2 extends within the cover 17 over a distance c which equals, or as shown, is less than a. Parts of the elements in the first embodiment shown in Fig. 1 can also be used in the second embodiment shown in Fig. 2 for example the use of optical adhesive 16 for securing the optical fibres 15.

With the embodiment of the device according to the invention shown in Fig. 2, the corona effect is achieved in the following way. Light 19 is transmitted from an external light source through the optical fibres 15 and to the tip 14 of the optical fibres 15. The light 19 is output forwards through the tip 14 of the optical fibres 15. The light 19 is directed through the space 21 or as in the shown embodiment through the translucent element 23 forwards towards the inner side 22 of the cover 17 and sideways towards the side surfaces 18 of the translucent element 23. The light 19 is reflected primarily off the inner side 22 of the cover 17, but is also reflected inside the translucent element 23. The light 19 is then directed backwards through the translucent element 23, alternatively the space 21, towards the back side 25 of the translucent element 23. The light 19 is then directed through the translucent element 2 and emitted from the device through the back side 7 of the translucent element 2. The light 19 emitted through the back side 7 of the translucent element 2 will provide a corona effect for the shape of the cover 17 in a plane perpendicular to the plane of the paper.

The device according to the invention has a depth d preferably of between 10 and 20 mm, but which may be more or less. The translucent element 1, the translucent element 2 and the translucent element 3 are made from acrylic, but other translucent materials may be used. The translucent element 3 which constitutes a reflecting element is preferably made from diffusion acrylic in which upwards of 80% of the light is reflected off the back side 9 of the translucent element 3 and the remaining part of the light is reflected inside the translucent element 3. The depth of the individual translucent elements may be arbitrary and the translucent element 1, the translucent element 2 and the translucent element 3 may be mutually fastened in other ways than by co-polymerisation.

Fig. 3 shows a sign for the facade of a building. The sign is the letter B viewed from the back and is made as a device according to the invention. The Figure shows the back side 7 of the translucent element 2 which has a pyramid structure and the optical fibres 15 extending inwards in the sign through the translucent element 2 and into the translucent element 1. Holes 12 for fitting more optical fibres 15 is also shown. On the optical fibres 15 are fitted sleeves 20. The sleeves 20 are intended to create a distance between the sign and the facade of the building when the sign is fastened to the facade of the building. Thus, light emitted through the back side 7 of the translucent element 2 will provide a corona effect around the sign and on the facade of the building. Alternatively to fitting the optical fibres 15 by means of optical adhesive 16, it will be possible to use the sleeves 20 as a connecting link between the optical fibres and the translucent element 2 alternatively the translucent element 1 or 23, respectively. The sleeves 20 are then capable of securing the optical fibres 15 and the sleeves 20 are then secured to the respective translucent element.

## Claims

1. A device for lighting, said device comprising a translucent element (2) and a reflector (17) situated at a distance from the translucent element (2), and wherein a light source is intended to send light (19) into a space (21) between the translucent element (2) and an inner side (22) of the reflector (17) **characterised in that** the light (19) is intended to be sent forwards towards the inner side (22) of the reflector (17), that the light is intended to be reflected off the inner side (22) of the reflector (17) and to be emitted through the back side (7) of the translucent element (2).

2. A device for lighting, said device comprising a translucent element (1,23) and a reflector (17) contacting the translucent element (1,23) and wherein a light source is intended to send light (19) into the translucent element (1,23), and wherein the light (19) is then intended to be reflected off the reflector (17) and to be sent out through the translucent element (1,23) **characterised in that** the translucent element (1,23) has a front side (4,24), a back side (5,25) and side surfaces (18,26), that the reflector (17) is in contact with the front side (4,24), that the light (19) is intended to be sent forwards towards the front side (4,24) of the translucent element (1,23), that the light (19) is intended to be reflected off the reflector (17) and to be emitted through the back side (5,25) of the translucent element (1,23).

3. A device according to claim 1 **characterised in that** the device at least comprises a first translucent element (1) and a second translucent element (2), that the first translucent element (1) comprises the front side (4) and the back side (5), that the front side (4) and the back side (5) are plane, that the reflector (17) is in contact with the front side of the first translucent element (1), that a front side (6) of the second translucent element (2) is in contact with the back side (5) of the first translucent element (1), and that the back side (7) of the second translucent element (2) has a non-plane surface.

4. A device according to claim 2 or claim 3 **characterised in that** the device at least comprises a first translucent element (1) and a third translucent element (3), that the first translucent element (1) comprises a front side (4) and a back side (5), that the front side (4) and the back side (5) are plane, that a back side (9) of the third translucent element (3) contacts the front side (4) of the first translucent element (1), and that the reflector (17) is in contact with the front side (8) of the third translucent element.

5. A device according to any one of the claims 3 and 4 **characterised in that** the translucent elements (1,2,3,23) are made from acrylic, and that the non-plane surfaces have pyramid structures.

6. A device according to any one of the claims 3 and 4 **characterised in that** the first translucent element (1) and the second translucent element (2), alternatively the first translucent element (1) and the third translucent element (3), are in contact with each other and secured to each other by means of co-polymerization, preferably by means of a layer of polymetyl methacrylate (PMMA).

7. A device according to any one of the preceding claims **characterised in that** a light (19) is transmitted to the device through an optical fibre (15), that a tip (14) of the optical fibre (15) is fitted in the first translucent element (1,23), and that the tip (14) of the optical fibre (15) is directed towards the front (4,24) of the first translucent element (1,23).

8. A device according to claim 7 **characterised in that** the tip (14) is fitted in a hole (12) in the first translucent element (1), and that the tip (14) of the optical fibre (15) is secured to the bottom (13) of the hole (12) by means of optical adhesive.

9. A device according to any one of the preceding claims 3-8 **characterised in that** a cover (17) of a non-translucent material covers the front side (4,24) and the side surfaces (18,26) of at least the first translucent element (1,23), said side surfaces (18,26) of the translucent element (1,23) being placed with a mutual distance a, and that the second translucent element extends outside the sides over the distance a.

10. Use of device according to any one of the preceding claims for making lighting with corona effect.

11. A device according to any of the preceding claims **characterised in that** the translucent element (2) extends beyond the periphery of the reflector (17).

## Patentansprüche

1. Beleuchtungseinrichtung, welche Einrichtung ein durchscheinendes Element (2) und einen Reflektor (17) aufweist, der sich in einem Abstand von dem durchscheinenden Element (2) befindet, und bei der eine Lichtquelle dazu bestimmt ist, Licht (19) in einen Raum (21) zwischen dem durchscheinenden Element (2) und einer Innenseite (22) des Reflektors (17) zu senden, **dadurch gekennzeichnet, dass** das Licht (19) dazu bestimmt ist, nach vorne in Richtung auf die Innenseite (22) des Reflektors (17) gesendet zu werden, und dass das Licht dazu bestimmt ist, von der Innenseite (22) des Reflektors (17) reflektiert und durch die Rückseite (7) des durchscheinenden Elements (2) hindurch ausgestrahlt zu werden.

2. Beleuchtungseinrichtung, welche Einrichtung ein durchscheinendes Element (1, 23) und einen Reflektor (17) aufweist, der das durchscheinende Element (1, 23) berührt, und bei der eine Lichtquelle dazu bestimmt ist, Licht (19) in das durchscheinende Element (1, 23) zu senden, und bei der das Licht (19) dann dazu bestimmt ist, von dem Reflektor (17) reflektiert und durch das durchscheinende Element (1, 23) hindurch ausgesendet zu werden, **dadurch gekennzeichnet, dass** das durchscheinende Element (1, 23) eine Vorderseite (4, 24), eine Rückseite (5, 25) und Seitenflächen (18, 26) hat, dass der Reflektor (17) die Vorderseite (4, 24) berührt, dass das Licht (19) dazu bestimmt ist, nach vorne in Richtung auf die Vorderseite (4, 24) des durchscheinenden Elements (1, 23) gesendet zu werden, und dass das Licht (19) dazu bestimmt ist, von dem Reflektor (17) reflektiert und durch die Rückseite (5, 25) des durchscheinenden Elements (1, 23) hindurch ausgestrahlt zu werden.

3. Einrichtung nach Anspruch 1, **dadurch gekennzeichnet, dass** die Einrichtung mindestens ein erstes durchscheinendes Element (1) und ein zweites durchscheinendes Element (2) aufweist, dass das erste durchscheinende Element (1) die Vorderseite (4) und die Rückseite (5) aufweist, dass die Vorderseite (4) und die Rückseite (5) eben sind, dass der Reflektor (17) die Vorderseite des ersten durchscheinenden Elements (1) berührt, dass eine Vorderseite (6) des zweiten durchscheinenden Elements (2) die Rückseite (5) des ersten durchscheinenden Elements (1) berührt und dass die Rückseite (7) des zweiten durchscheinenden Elements (2) eine nicht ebene Oberfläche hat.

4. Einrichtung nach Anspruch 2 oder Anspruch 3, **dadurch gekennzeichnet, dass** die Einrichtung mindestens ein erstes durchscheinendes Element (1) und ein drittes durchscheinendes Element (3) aufweist, dass das erste durchscheinende Element (1) eine Vorderseite (4) und eine Rückseite (5) aufweist, dass die Vorderseite (4) und die Rückseite (5) eben sind, dass eine Rückseite (9) des dritten durchscheinenden Elements (3) die Vorderseite (4) des ersten durchscheinenden Elements (1) berührt und dass der Reflektor (17) die Vorderseite (8) des dritten durchscheinenden Elements berührt.

5. Einrichtung nach einem der Ansprüche 3 und 4, **dadurch gekennzeichnet, dass** die durchscheinenden Elemente (1, 2, 3, 23) aus Acryl bestehen und dass die nicht ebenen Oberflächen Pyramidenstrukturen haben.

6. Einrichtung nach einem der Ansprüche 3 und 4, **dadurch gekennzeichnet, dass** das erste durchscheinende Element (1) und das zweite durchscheinende Element (2), alternativ das erste durchscheinende Element (1) und das dritte durchscheinende Element (3), einander berühren und mittels Copolymerisation aneinander befestigt sind, vorzugsweise mittels einer Schicht Polymethylmethacrylat (PMMA).

7. Einrichtung nach einem der vorhergehenden Ansprüche, **dadurch gekennzeichnet, dass** Licht (19) durch einen Lichtwellenleiter (15) auf die Einrichtung übertragen wird, dass eine Spitze (14) des Lichtwellenleiters (15) in das erste durchscheinende Element (1, 23) eingefügt ist und dass die Spitze (14) des Lichtwellenleiters (15) zur Vorderseite (4, 24) des ersten durchscheinenden Elements (1) hin gerichtet ist.

8. Einrichtung nach Anspruch 7, **dadurch gekennzeichnet, dass** die Spitze (14) in ein Loch (12) im ersten durchscheinenden Element (1) eingefügt ist und dass die Spitze (14) des Lichtwellenleiters (15) mittels optischem Klebstoff am Boden (13) des Lochs (12) befestigt ist.

9. Einrichtung nach einem der vorhergehenden Ansprüche 3 bis 8, **dadurch gekennzeichnet, dass** eine Abdeckung (17) aus einem nicht durchscheinenden Material die Vorderseite (4, 24) und die Seitenflächen (18, 26) mindestens des ersten durchscheinenden Elements (1, 23) bedeckt, dass die Seitenflächen (18, 26) des durchscheinenden Elements (1, 23) in einer Distanz a voneinander angeordnet sind und dass sich das zweite durchscheinende Element außerhalb der Seiten über die Distanz a erstreckt.

10. Verwendung einer Einrichtung nach einem der vorhergehenden Ansprüche zur Herstellung von Beleuchtung mit Koronaeffekt.

11. Einrichtung nach einem der vorhergehenden Ansprüche, **dadurch gekennzeichnet, dass** sich das durchscheinende Element (2) über den Umfang des Reflektors (17) hinaus erstreckt.

## Revendications

1. Dispositif d'éclairage, ce dispositif comprenant un élément translucide (2) et un réflecteur (17) situé à distance de l'élément translucide (2), et dans lequel une source de lumière est destinée à envoyer de la lumière (19) dans un espace (21) situé entre l'élément translucide (2) et une face interne (22) du réflecteur (17), **caractérisé en ce que** la lumière (19) est destinée à être envoyée vers l'avant, en direction de la face interne (22) du réflecteur (17), **en ce que** la lumière est destinée à être réfléchie par la face interne (22) du réflecteur (17) et à être émise à travers la face arrière (7) de l'élément translucide (2).

2. Dispositif d'éclairage, ce dispositif comprenant un élément translucide (1, 23) et un réflecteur (17) contactant l'élément translucide (1, 23) et dans lequel une source de lumière est destinée à envoyer de la lumière (19) dans l'élément translucide (1, 23), et dans lequel la lumière (19) est ensuite destinée à être réfléchie par le réflecteur (17) et à être émise à travers l'élément translucide (1, 23), **caractérisé en ce que** l'élément translucide (1, 23) possède une face avant (4, 24), une face arrière (5, 25) et des surfaces latérales (18, 26), **en ce que** le réflecteur (17) est en contact avec la face avant (4, 24), **en ce que** la lumière (19) est destinée à être envoyée vers l'avant, en direction de la face avant (4, 24) de l'élément translucide (1, 23), **en ce que** la lumière (19) est destinée à être réfléchie par le réflecteur (17) et à être émise à travers la face arrière (5, 25) de l'élément translucide (1, 23).

3. Dispositif selon la revendication 1, **caractérisé en ce que** le dispositif comprend au moins un premier élément translucide (1) et un deuxième élément translucide (2), **en ce que** le premier élément translucide (1) comprend la face avant (4) et la face arrière (5), **en ce que** la face avant (4) et la face arrière (5) sont planes, **en ce que** le réflecteur (17) est en contact avec la face avant du premier élément translucide (1), **en ce qu'**une face avant (6) du deuxième élément translucide (2) est en contact avec la face arrière (5) du premier élément translucide (1), et **en ce que** la face arrière (7) du deuxième élément translucide (2) a une surface qui n'est pas plane.

4. Dispositif selon la revendication 2 ou la revendication 3, **caractérisé en ce que** le dispositif comprend au moins un premier élément translucide (1) et un troisième élément translucide (3), **en ce que** le premier élément translucide (1) comprend une face avant (4) et une face arrière (5), **en ce que** la face avant (4) et la face arrière (5) sont planes, **en ce qu'**une face arrière (9) du troisième élément translucide (3) est en contact avec la face avant (4) du premier élément translucide (1), et **en ce que** le réflecteur (17) est en contact avec la face avant (8) du troisième élément translucide.

5. Dispositif selon l'une quelconque des revendications 3 et 4, **caractérisé en ce que** les éléments translucides (1, 2, 3, 23) sont constitués en acrylique, et **en ce que** les surfaces qui ne sont pas planes présentent des structures pyramidales.

6. Dispositif selon l'une quelconque des revendications 3 et 4, **caractérisé en ce que** le premier élément translucide (1) et le deuxième élément translucide (2) ou, en alternative, le premier élément translucide (1) et le troisième élément translucide (3), sont en contact l'un avec l'autre et fixés l'un à l'autre par copolymérisation, de préférence au moyen d'une couche de polyméthyl-méthacrylate (PMMA).

7. Dispositif selon l'une quelconque des revendications précédentes, **caractérisé en ce qu'**une lumière (19) est transmise au dispositif par une fibre optique (15), **en ce qu'**un bout (14) de la fibre optique (15) est inséré dans le premier élément translucide (1, 23), et **en ce que** le bout (14) de la fibre optique (15) est dirigé en direction de l'avant (4, 24) du premier élément translucide (1, 23).

8. Dispositif selon la revendication 7, **caractérisé en ce que** le bout (14) est inséré dans un trou (12) ménagé dans le premier élément translucide (1), et **en ce que** le bout (14) de la fibre optique (15) est fixé au fond (13) du trou (12) par un adhésif optique.

9. Dispositif selon l'une quelconque des revendications 3-8, **caractérisé en ce qu'**un couvercle (17) en matériau non-translucide recouvre la face avant (4, 24) et les surfaces latérales (18, 26) d'au moins le premier élément translucide (1, 23), ces surfaces latérales (18, 26) de l'élément translucide (1, 23) étant disposées à une distance a l'une de l'autre, et **en ce que** le deuxième élément translucide s'étend sur la distance a, en dépassant des côtés.

10. Utilisation du dispositif selon l'une quelconque des revendications précédentes pour produire un éclairage à effet corona.

11. Dispositif selon l'une quelconque des revendications précédentes, **caractérisé en ce que** l'élément translucide (2) s'étend au-delà de la périphérie du réflecteur (17).
